# FASCICULE DE BREVET EUROPEEN

(11) **EP 0 984 787 B1**
(45) Date de publication et mention de la délivrance du brevet: **02.10.2002**
(21) Numéro de dépôt: 98925774.6
(22) Date de dépôt: 25.05.1998
(51) Int. Cl.: A61K 31/727, A61P 7/10

(54) **UTILISATION DES HEPARINES DE BAS POIDS MOLECULAIRE POUR LA PREVENTION ET LE TRAITEMENT DES OEDEMES CEREBRAUX**
VERWENDUNG VON HEPARINEN NIEDRIGEREN MOLEKULARGEWICHTES ZUR BEHANDLUNG VON HIRNÖDEMEN
USE OF LOW-MOLECULAR-WEIGHT HEPARINS FOR PREVENTING AND TREATING CEREBRAL EDEMAS

(30) Priorité: 28.05.1997 FR 9706551
(43) Date de publication de la demande: 15.03.2000
(73) Titulaire: Aventis Pharma S.A., 92160 Antony (FR)
(72) Inventeur: MARY, Véronique, F-91000 Evry (FR); PRATT, Jérémy, F-94600 Choisy le Roi (FR); STUTZMANN, Jean-Marie, F-94440 Villecresnes (FR); UZAN, André, F-75116 Paris (FR); WAHL, Florence, F-75004 Paris (FR)
(86) Numéro de dépôt international: FR9801035
(87) Numéro de publication internationale: WO98053834

(56) Documents cités:
- B S ET AL.: "Stratégies thérapeutiques médicales de l'ischémie cérébrale aiguë de l'adulte." J. NEURORADIOL., vol. 20, no. 2, 1993, pages 102-120, XP002053395
- VAN GIJN J.: "Thrombolysis in Ischemic Stroke: Double or Quits?" CIRCULATION, vol. 93, no. 9, 1996, pages 1616-1617, XP002053396
- MARX P.: "Die Therapie des akuten ischämischen Insultes" HÄMOSTASEOLOGIE, vol. 17, no. 2, 1997, pages 86-91, XP002053397
- DATABASE WPI Section Ch, Week 9506 Derwent Publications Ltd., London, GB; Class B04, AN 95-042020 XP002053404 & RU 2 013 092 C (DAGESTAN MED INST) , 30 mai 1994
- FAREED ET AL.: "Comparative study on the in vitro and in vivo Activities of Seven Low-Molecular-Weight Heparins" HAEMOSTASIS, vol. 18, no. suppl. 3, 1988, pages 3-15, XP002053398
- FUJII ET AL.: "Treatment for Delayed Brain Injury after Pituitary Irradiation" NEUROL. SURG., vol. 16, no. 3, 1988, pages 241-247, XP002053399
- GREEN ET AL.: "Prevention of Thromboembolism after Spinal Cord Injury Using Low-Molecular-Weight Heparin" ANN. INTERN. MED., vol. 113, 1990, pages 571-574, XP002053400
- KAY ET AL.: "Low-molecular-weight heparin for the treatment of acute ischemic stroke" N. ENGL. J. MED., vol. 333, 1995, pages 1588-1593, XP002053401
- DI STEFANO ET AL.: "Low molecular weight heparins for long-term therapy of peripheral vascular disease." CURR. THER. RES. CLIN. EXP., vol. 44, no. 1, 1988, pages 1-10, XP002053402
- TZONOS ET AL.: "Die Wirkung von Proteinasen und Fibrinolyse-Inhibitoren auf das experimentelle Hirnödem" Z. NEUROL., vol. 205, no. 1, 1973, pages 61-70, XP002053403

## Description

La présente invention concerne l'utilisation des héparines de bas poids moléculaire pour la prévention et le traitement des oedèmes cérébraux.

L'invention concerne également l'utilisation des héparines de bas poids moléculaire pour la préparation d'un médicament utile pour la prévention et le traitement des oedèmes cérébraux.

L'héparine standard est un polysaccharide sulfaté de poids moléculaire moyen de 12000-15000 daltons isolé des muqueuses intestinales de boeuf, de mouton et de porc. L'héparine est utilisée cliniquement pour la prévention et le traitement des désordres thromboemboliques mais cause parfois des hémorragies.

Depuis une dizaine d'années, l'héparine est progressivement remplacée par les héparines de bas poids moléculaire qui ne présentent plus ou à un degré moindre l'inconvénient de faire saigner et ne nécessitent plus qu'une injection par jour au lieu de 2 à 3 injections par jour pour l'héparine standard. Ces héparines de bas poids moléculaire sont préparées notamment par fractionnement, dépolymérisation controlée de l'héparine ou par synthèse chimique. Elles présentent un rapport activité anti Xa/activité anti IIa supérieur à 2.

Il a maintenant été trouvé que les héparines de bas poids moléculaire réduisent les oedèmes cérébraux.

Les oedèmes cérébraux représentent une conséquence aggravante majeure des accidents vasculaires cérébraux thromboemboliques, des hémorragies cérébrales et des traumatismes du système nerveux central (traumatisme cérébral et trauma médullaire) mais également des arrêts cardiaques et/ou respiratoires, ou toute situation fonctionnellement équivalente quelle qu'en soit l'origine (fibrillation ventriculaire, crise d'asthme maligne). Les oedèmes cérébraux peuvent également se rencontrer dans toute situation pathologique ou accidentelle de coma accompagnés d'une hypoperfusion du parenchyme cérébral. Les oedèmes cérébraux peuvent aussi accompagner les tumeurs cérébrales, les irradiations et des infections bactériennes, virales et parasitaires du parenchyme cérébral.

Selon l'invention, on utilise de préférence une héparine de bas poids moléculaire ayant un poids moléculaire moyen compris entre 1000 et 10000 daltons, notamment entre 1500 et 6000 daltons et, en particulier, entre 4000 et 5000 daltons.

Elles peuvent être préparées par différents procédés à partir de l'héparine :
- fractionnement au moyen de solvants (FR2440376, US 4692435),
- fractionnement sur résine anionique (FR2453875),
- gel filtration (BARROWCLIFFE, Thromb. Res. 12, 27-36 (1977),
- Chromatographie d'affinité (US4401758),
- dépolymérisation contrôlée au moyen d'un agent chimique : acide nitreux (EP14184, EP37319, EP76279, EP623629, FR2503714, US4804652; WO813276), β élimination à partir d'un ester de l'héparine (EP40144, US5389618), périodate (EP287477), borohydrure de sodium (EP347588, EP380943), acide ascorbique (US 4533549); peroxyde d'hydrogène (US4629699, US4791195), hydroxyde d'ammonium quaternaire à partir d'un sel d'ammonium quaternaire d'héparine (US4981955), hydroxyde de métal alcalin (EP380943, EP347588) ou par voie enzymatique (EP64452, US4396762, EP244235, EP244236; US 4826827; US 3766167); au moyen d'irradiation (EP 269981).

Certaines peuvent également être préparées par synthèse chimique (US4801583, US4818816, EP165134, EP84999, FR2535306)

Parmi ces héparines de bas poids moléculaire, on peut citer plus particulièrement l'énoxaparine (DCI) commercialisée par RHONE-POULENC RORER, la nadroparine (DCI) commercialisée par SANOFI, la parnaparine (DCI) commercialisée par OPOCRIN-ALFA, la reviparine (DCI) commercialisée par KNOLL, la daltéparine (DCI) commercialisée par KABI PHARMACIA, la tinzaparine (DCI) commercialisée par NOVO NORDISK, la danaparoide (DCI) commercialisée par ORGANON, l'ardeparine (DCI) développée par WYETH AYERST, la certoparine (DCI) commercialisée par SANDOZ et les produits en étude tels que le CY222 de SANOFI-CHOAY (Thromb. Haemostasis, 58 (1), 553 (1987)), le SR90107/ORG31540 de SANOFI-ORGANON (Thrombosis and Haemostasis, 74, 1468-1473 (1995)).

Préférentiellement, les héparines de bas poids moléculaire sont constituées d'oligosaccharides ayant un acide 2-O-sulfo-4-énopyranosuronique à l'une de leurs extrêmités.

Une héparine de bas poids moléculaire particulièrement avantageuse est obtenue par dépolymérisation d'un ester de l'héparine au moyen d'une base telle que la soude.

A - L'action préventive des héparines de bas poids moléculaire sur l'oedème a été déterminée chez le rat après lésion cérébrale photothrombotique selon le protocole suivant : des rats mâles Sprague Dawley (150-200g) (26 pour le groupe traité et 22 pour le groupe témoin) sont anesthésiés avec du chloral hydrate (400 mg/kg ip) et placés en contention stéréotaxique. La peau est incisée pour révéler le crâne et une lumière froide (Bioblock 150W) est mise en contact avec le côté droit du crâne devant le lambda. Les héparines de bas poids moléculaire sont dissoutes dans une solution saline (NaCl 0,9%) et et on poursuit de la manière suivante :

3 heures avant la lésion, on injecte, par voie sous cutanée, 2 mg/kg/5ml de l'héparine de bas poids moléculaire,
juste avant la lésion, on injecte par voie intraveineuse, 0,5mg/kg/5ml de l'héparine de bas poids moléculaire et immédiatement après, par voie sous cutanée, 10mg/kg/5ml de colorant rose bengale dans une solution saline (0,9% NaCI),
la lésion est ensuite effectuée par illumination du crâne pendant 5 minutes puis la peau est suturée et les animaux sont remis dans leurs cages,
3 heures et 21 heures après la lésion, on injecte, par voie sous cutanée, 2mg/kg/5ml de l'héparine de bas poids moléculaire,
24 heures après la lésion, les animaux sont décapités et les cerveaux sont récupérés.

Des échantillons sont récueillis au site de la lésion et contralatéralement à la lésion en utilisant un foret en liège de 6 mm de diamètre. Le volume d'eau des échantillons est déterminé par le rapport poids humide du tissu/ poids sec du tissu et l'oedème exprimé comme le pourcentage d'eau en excès sur l'échantillon lésioné comparé à l'échantillon de l'hémisphère contralatéral pour chaque rat. Les animaux témoins reçoivent du sérum physiologique dans les mêmes conditions.

Dans ce test, les héparines de bas poids moléculaire réduisent l'oedème d'environ 30%.

L'énoxaparine réduit l'oedème de 33%.

Ces résultats démontrent que les héparines de bas poids moléculaire ont un effet préventif sur l'oedème.

B - L'action curative des héparines de bas poids moléculaire sur l'oedème a été déterminée chez le rat après lésion cérébrale photothrombotique selon le protocole suivant : des rats mâles Sprague Dawley (200-240g) (20 pour le groupe témoin et 12 et 8 pour les groupes traités) sont anesthésiés avec du chloral hydrate (400 mg/kg ip) et placés en contention stéréotaxique. La peau est incisée pour révéler le crâne et une lumière froide (Bioblock 150W) est mise en contact avec le côté droit du crâne devant le lambda. On injecte, par voie intraveineuse, 10mg/kg/5ml de colorant rose bengale dans du sérum physiologique. Le crâne est ensuite éclairé pendant 5 minutes. La peau est ensuite suturée. Les héparines de bas poids moléculaire sont dissoutes dans une solution saline (NaCl 0,9%) puis, soit 2 heures après la lésion, on injecte par voie intraveineuse 0,5mg/kg/5ml de l'héparine de bas poids moléculaire puis 15 minutes après, par voie sous cutanée, 2mg/kg/5ml de l'héparine de bas poids moléculaire, soit 6 heures après la lésion on injecte, par voie sous cutanée, 2 mg/kg/5ml de l'héparine de bas poids moléculaire. Les animaux sont remis dans leurs cages. 24 heures après la lésion, les animaux sont décapités et les cerveaux sont récupérés. Des échantillons sont récueillis au site de la lésion et contralatéralement à la lésion en utilisant un foret en liège de 6 mm de diamètre. Le volume d'eau est déterminé par le rapport poids humide du tissu/ poids sec du tissu et l'oedème exprimé comme le pourcentage d'eau en excès sur l'échantillon lésioné comparé à l'échantillon de l'hémisphère contralatéral pour chaque rat. Les animaux témoins reçoivent du sérum physiologique dans les mêmes conditions.

Dans ce test, les héparines de bas poids moléculaire réduisent l'oedème d'environ 30% lorsque l'héparine de bas poids moléculaire est injectée 2 heures ou 6 heures après la lésion.

L'énoxaparine réduit l'oedème de 33% dans les 2 cas.

Dans le même test mais dans lequel les héparines de bas poids moléculaire sont injectées 18 heures après la lésion (0,5mg/kg/5ml par voie intraveineuse puis 15 minutes plus tard 2 mg/kg/5 ml par voie sous cutanée), la réduction de l'oedème est encore significative puisqu'elle est d'environ 10 à 30% (énoxaparine 23% et nadroparine 14%).

Ces résultats démontrent que les héparines de bas poids moléculaire ont un effet curatif sur l'oedème.

C - L'effet des héparines de bas poids moléculaire sur l'oedème cérébral est également démontré chez le rat sur l'oedème induit par un trauma selon la technique suivante : des rats mâles Sprague-Dawley (Charles River France) pesant 280-300 g (12 pour le groupe témoin et 12 pour le groupe traité) sont anesthésiés à l'halothane (1,5%) dans un mélange N₂O/O₂ (70/30) et placés en contention stéréotaxique. L'épicrâne est incisé et un trou est effectué au moyen d'une perceuse dentée au niveau du cortex pariétal droit (coordonnées : 3,5 mm antérieur à 6 mm au-dessus de la ligne interaurale). Un tube de polyéthylène de 3 mm de diamètre intérieur est placé sur la dure mère, fixé dans la cavité crânienne avec du ciment dentaire et relié à une valve solénoïde 5Danfoss Evsi 24v, 15W). La dure mère est gardée intacte. La valve est reliée à une pompe HPLC (Walters 590). Le système est rempli d'eau stérile et quand la pompe a atteint une pression de 3,8 à 4 bar, la percussion fluide de sévérité modérée (1,6-1,8 bar) est induite par une brève ouverture (20 ms) de la valve. Le tube est ensuite retiré, l'incision suturée et les animaux sont remis dans leur cage dans une pièce chauffée à 26-28°C.

Les héparines de bas poids moléculaire dissoutes dans une solution saline (NaCI 0,9%) sont administrées de la manière suivante : 2 heures après la lésion : 0,5mg/kg/5ml IV bolus, 2 heures 15 minutes après la lésion 2mg/kg/5ml SC, 6 heures après la lésion : 2 mg/kg/5ml SC, 24 heures après la lésion : 2 mg/kg/5ml SC et 30 heures après la lésion : 2 mg/kg/5ml SC.

Au groupe témoin on administre 5 ml/kg d'une solution saline (NaCl 0,9%) dans les mêmes conditions.

Les animaux sont sacrifiés 48 heures après la lésion. L'oedème cérébral est évalué selon la technique poids humide/poids sec (24 heures à 100°C).

L'oedème évalué comme la teneur en eau du cerveau est mesuré dans l'hippocampe et le cortex adjacent au site de lésion.

Dans ce test, les héparines de bas poids moléculaire réduisent d'au moins 40% l'oedème dans l'hippocampe et dans le cortex adjacent au site de lésion.

L'énoxaparine (LOVENOX^{R}) réduit l'oedème de 69% dans l'hippocampe et de 50% dans le cortex adjacent au site de lésion.

Les médicaments sont constitués par un sel (sodium ou calcium de préférence) d'une héparine de bas poids moléculaire sous forme d'une composition dans laquelle il est associé à tout autre produit pharmaceutiquement compatible, pouvant être inerte ou physiologiquement actif. Les médicaments selon l'invention peuvent être employés par voie intraveineuse, sous-cutanée, orale, rectale, topique ou pulmonaire (inhalation).

Les compositions stériles pour administration intraveineuse ou sous-cutanée, sont généralement des solutions aqueuses. Ces compositions peuvent également contenir des adjuvants, en particulier des agents mouillants, isotonisants, émulsifiants, dispersants et stabilisants. La stérilisation peut se faire de plusieurs façons, par exemple par filtration aseptisante, en incorporant à la composition des agents stérilisants, par irradiation. Elles peuvent également être préparées sous forme de compositions solides stériles qui peuvent être dissoutes au moment de l'emploi dans de l'eau stérile ou tout autre milieu stérile injectable.

Comme compositions solides pour administration orale, peuvent être utilisés des comprimés, des pilules, des poudres (capsules de gélatine, cachets) ou des granulés. Dans ces compositions, le principe actif est mélangé à un ou plusieurs diluants inertes, tels que amidon, cellulose, saccharose, lactose ou silice, sous courant d'argon. Ces compositions peuvent également comprendre des substances autres que les diluants, par exemple un ou plusieurs lubrifiants tels que le stéarate de magnésium ou le talc, un agent favorisant l'absorption orale, un colorant, un enrobage (dragées) ou un vernis.

Comme compositions liquides pour administration orale, on peut utiliser des solutions, des suspensions, des émulsions, des sirops et des élixirs pharmaceutiquement acceptables contenant des diluants inertes tels que l'eau, l'éthanol, le glycérol, les huiles végétales ou l'huile de paraffine. Ces compositions peuvent comprendre des substances autres que les diluants, par exemple des produits mouillants, édulcorants, épaississants, aromatisants ou stabilisants.

Les compositions pour administration rectale sont les suppositoires ou les capsules rectales qui contiennent, outre le produit actif, des excipients tels que le beurre de cacao, des glycérides semi-synthétiques ou des polyéthylèneglycols.

Les compositions pour administration topique peuvent être par exemple des crèmes, lotions, collyres, collutoires, gouttes nasales ou aérosols.

Les doses dépendent de l'effet recherché, de la durée du traitement et de la voie d'administration utilisée; elles sont généralement comprises entre 0,2 mg et 4 mg par kg par jour par voie sous-cutanée soit 14 à 280 mg par jour pour un adulte.

D'une façon générale, le médecin déterminera la posologie appropriée en fonction de l'âge, du poids et de tous les autres facteurs propres au sujet à traiter.

La présente invention concerne également l'utilisation pour la prévention ou le traitement des oedèmes cérébraux chez l'homme comprenant l'administration d'une quantité efficace d'une héparine de bas poids moléculaire.

## Revendications

1. Utilisation d'une héparine de bas poids moléculaire pour la préparation d'un médicament utile pour la prévention et le traitement des oedèmes cérébraux.

2. Utilisation selon la revendication 1 pour laquelle l'héparine de bas poids moléculaire a un poids moléculaire moyen compris entre 1000 et 10000 daltons.

3. Utilisation selon la revendication 1 pour laquelle l'héparine de bas poids moléculaire a un poids moléculaire moyen compris entre 1500 et 6000 daltons.

4. Utilisation selon la revendication 1 pour laquelle l'héparine de bas poids moléculaire a un poids moléculaire moyen compris entre 4000 et 5000 daltons.

5. Utilisation selon l'une des revendications 1 à 4 pour laquelle l'héparine de bas poids moléculaire est constituée d'oligosaccharides ayant un acide 2-O-sulfo-4-énopyranosuronique à l'une de leurs extrémités.

6. Utilisation selon l'une des revendications 1 à 5 pour laquelle l'héparine de bas poids moléculaire est obtenue par dépolymérisation d'un ester de l'héparine au moyen d'une base.

7. Utilisation selon l'une des revendications 1 à 6 pour laquelle l'héparine de bas poids moléculaire est l'énoxaparine (DCI).

8. Utilisation selon l'une des revendications 1 à 4 pour laquelle l'héparine de bas poids moléculaire la nadroparine (DCI).

9. Utilisation selon l'une des revendications 1 à 4 pour laquelle l'héparine de bas poids moléculaire est la parnaparine (DCI).

10. Utilisation selon l'une des revendications 1 à 4 pour laquelle l'héparine de bas poids moléculaire est la reviparine (DCI).

11. Utilisation selon l'une des revendications 1 à 4 pour laquelle l'héparine de bas poids moléculaire est la daltéparine (DCI).

12. Utilisation selon l'une des revendications 1 à 4 pour laquelle l'héparine de bas poids moléculaire est la tinzaparine (DCI).

13. Utilisation selon l'une des revendications 1 à 4 pour laquelle l'héparine de bas poids moléculaire la danaparoide (DCI).

14. Utilisation selon l'une des revendications 1 à 7 pour laquelle l'héparine de bas poids moléculaire est l'ardeparine (DCI).

15. Utilisation selon l'une des revendications 1 à 7 pour laquelle l'héparine de bas poids moléculaire est la certoparine (DCI).

16. Utilisation selon l'une des revendications 1 à 4 pour laquelle l'héparine de bas poids moléculaire est le CY222.

17. Utilisation selon l'une des revendications 1 à 4 pour laquelle l'héparine de bas poids moléculaire est le SR90107/ORG31540.

## Claims

1. Use of a low-molecular-weight heparin for the preparation of a medicament for the prevention and treatment of cerebral oedemas.

2. Use according to claim 1, for which the low-molecular-weight heparin has an average molecular weight of between 1000 and 10,000 daltons.

3. Use according to claim 1, for which the low-molecular-weight heparin has an average molecular weight of between 1500 and 6000 daltons.

4. Use according to claim 1, for which the low-molecular-weight heparin has an average molecular weight of between 4000 and 5000 daltons.

5. Use according to one of claims 1 to 4, for which the low-molecular-weight heparin consists of oligosaccharides having a 2-O-sulpho-4-enopyranosuronic acid at one of their ends.

6. Use according to one of claims 1 to 5, for which the low-molecular-weight heparin is obtained by depolymerization of a heparin ester by means of a base.

7. Use according to one of claims 1 to 6, for which the low-molecular-weight heparin is enoxaparin (INN).

8. Use according to one of claims 1 to 4, for which the low-molecular-weight heparin is nadroparin (INN).

9. Use according to one of claims 1 to 4, for which the low-molecular-weight heparin is parnaparin (INN).

10. Use according to one of claims 1 to 4, for which the low-molecular-weight heparin is reviparin (INN).

11. Use according to one of claims 1 to 4, for which the low-molecular-weight heparin is dalteparin (INN).

12. Use according to one of claims 1 to 4, for which the low-molecular-weight heparin is tinzaparin (INN).

13. Use according to one of claims 1 to 4, for which the low-molecular-weight heparin is danaparoid (INN).

14. Use according to one of claims 1 to 7, for which the low-molecular-weight heparin is ardeparin (INN).

15. Use according to one of claims 1 to 7, for which the low-molecular-weight heparin is certoparin (INN).

16. Use according to one of claims 1 to 4, for which the low-molecular-weight heparin is CY222.

17. Use according to one of claims 1 to 4, for which the low-molecular-weight heparin is SR90107/ORG31540.

## Patentansprüche

1. Verwendung eines Heparins mit niedrigem Molekulargewicht für die Herstellung eines Arzneimittels zur Prävention und Behandlung von cerebralen Ödemen.

2. Verwendung nach Anspruch 1, für die das Heparin mit niedrigem Molekulargewicht ein mittleres Molekulargewicht zwischen 1000 und 10 000 Dalton hat.

3. Verwendung nach Anspruch 1, für die das Heparin mit niedrigem Molekulargewicht ein mittleres Molekulargewicht zwischen 1500 und 6000 Dalton hat.

4. Verwendung nach Anspruch 1, für die das Heparin mit niedrigem Molekulargewicht ein mittleres Molekulargewicht zwischen 4000 und 5000 Dalton hat.

5. Verwendung nach einem der Ansprüche 1 bis 4, für die das Heparin mit niedrigem Molekulargewicht aus Oligosacchariden besteht, die an einem ihrer Enden eine 2-O-Sulfo-4-enopyranosuronsäure haben.

6. Verwendung nach einem der Ansprüche 1 bis 5, für die das Heparin mit niedrigem Molekulargewicht durch Depolymerisation eines Esters des Heparins durch eine Base erhalten wird.

7. Verwendung nach einem der Ansprüche 1 bis 6, für die das Heparin mit niedrigem Molekulargewicht Enoxaparin (DCI) ist.

8. Verwendung nach einem der Ansprüche 1 bis 4, für die das Heparin mit niedrigem Molekulargewicht Nadroparin (DCI) ist.

9. Verwendung nach einem der Ansprüche 1 bis 4, für das Heparin mit niedrigem Molekulargewicht Parnaparin (DCI) ist.

10. Verwendung nach einem der Ansprüche 1 bis 4, für die das Heparin mit niedrigem Molekulargewicht Reviparin (DCI) ist.

11. Verwendung nach einem der Ansprüche 1 bis 4, für die das Heparin mit niedrigem Molekulargewicht Dalteparin (DCI) ist.

12. Verwendung nach einem der Ansprüche 1 bis 4, für die das Heparin mit niedrigem Molekulargewicht Tinzaparin (DCI) ist.

13. Verwendung nach einem der Ansprüche 1 bis 4, für die das Heparin mit niedrigem Molekulargewicht Danaparoid (DCI) ist.

14. Verwendung nach einem der Ansprüche 1 bis 7, für die das Heparin mit niedrigem Molekulargewicht Ardeparin (DCI) ist.

15. Verwendung nach einem der Ansprüche 1 bis 7, für die das Heparin mit niedrigem Molekulargewicht Certoparin (DCI) ist.

16. Verwendung nach einem der Ansprüche 1 bis 4, für die das Heparin mit niedrigem Molekulargewicht CY222 ist.

17. Verwendung nach einem der Ansprüche 1 bis 4, für die das Heparin mit niedrigem Molekulargewicht SR90107/ORG31540 ist.
